(19) Europäisches Patentamt
European Patent Office
Office européen des brevets

(11) **EP 3 485 870 B1**

(12) **FASCICULE DE BREVET EUROPEEN**

(45) Date de publication et mention
de la délivrance du brevet:
**08.03.2023   Bulletin 2023/10**

(51) Classification Internationale des Brevets (IPC):
***A61K 8/46*** *(2006.01)*       ***A61Q 19/00*** *(2006.01)*
***A61K 8/23*** *(2006.01)*

(21) Numéro de dépôt: **18198267.9**

(22) Date de dépôt: **02.10.2018**

(52) Classification Coopérative des Brevets (CPC):
**A61Q 19/00; A61K 8/23; A61K 8/466;**
A61K 2800/10; A61K 2800/52; A61K 2800/522;
A61K 2800/524

(54) **COMPOSITION COSMETIQUE STABILISANTE POUR REDUIRE LA DEGRADATION D'ACTIFS COSMETIQUES INSTABLES**

STABILISIERENDE KOSMETISCHE ZUSAMMENSETZUNG ZUR REDUKTION DES ABBAUS VON INSTABILEN KOSMETISCHEN AKTIVSTOFFEN

STABILISING COSMETIC COMPOSITION FOR REDUCING THE DEGRADATION OF UNSTABLE COSMETIC ACTIVE INGREDIENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **20.11.2017   FR 1760950**

(43) Date de publication de la demande:
**22.05.2019   Bulletin 2019/21**

(73) Titulaire: **Thorel, Jean-Noël**
**75014 Paris (FR)**

(72) Inventeur: **Thorel, Jean-Noël**
**75014 Paris (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A2- 1 166 780          WO-A1-2008/052674**
**DE-A1-102009 048 974     FR-A1- 3 007 652**
**US-A1- 2011 151 077**

## Description

### DOMAINE TECHNIQUE

La présente invention concerne une association d'antioxydants destinée à stabiliser au moins un actif contenu dans une composition cosmétique pour application topique. La présente invention concerne également un procédé de stabilisation d'actifs cosmétiques instables basé sur ladite association d'antioxydants.

### ETAT DE LA TECHNIQUE

Les formulations des produits cosmétiques contiennent généralement des ingrédients actifs destinés à favoriser la régénération de la peau, l'apaiser, la protéger contre le vieillissement ou les méfaits de la lumière ou encore soigner des conditions pathologiques de la peau. Une vaste partie des ingrédients actifs utilisés en cosmétique sont issus du monde végétal.

En particulier, on peut citer les polyphénols qui se retrouvent dans la plupart des produits cosmétiques, soit sous forme d'extraits, soit sous forme de molécules synthétiques ou purifiées. Ces composés ont été sélectionnés à travers les siècles pour les propriétés dermocosmétiques qu'ils confèrent aux compositions les contenant.

Cependant, il s'agit de composés souvent instables, qui ont tendance à s'oxyder ou se dégrader, par exemple par formation d'oligomères, s'ils sont exposés à la lumière, aux températures élevées ou à un environnement pro-oxydant (notamment, par mise en contact avec des ions métalliques).

La dégradation des actifs cosmétiques peut entraîner une modification de l'odeur, de la couleur, ou du toucher des compositions qui, bien que n'ayant pas forcément d'impact négatif sur l'efficacité du produit, ne sont pas acceptables pour les consommateurs. En effet, les utilisateurs des produits cosmétiques souhaitent disposer de produits qui sont stables et qui maintiennent leurs propriétés inaltérées pendant toute leur durée de vie.

Plusieurs stratégies ont été proposées pour stabiliser les actifs instables, notamment les composés phénoliques et plus particulièrement les polyphénols, dans les produits cosmétiques. A titre d'exemple, on peut citer le document FR 2 778 663, qui décrit des procédés de stabilisation des flavonoïdes par estérification et les composés issus de ces procédés.

Cependant, la modification chimique des actifs peut entraîner un problème de tolérance cutanée et expose le consommateur au risque lié à la toxicité des impuretés résiduelles présentes (intermédiaires et précurseurs chimiques).

L'acide éthylène diamine tétra-acétique (EDTA) est également utilisé de façon routinière pour stabiliser les compositions cosmétiques contenant des actifs susceptibles de se dégrader. L'EDTA est un chélateur et par cette action, il contribue à limiter l'oxydation induite par les ions métalliques.

Cependant, l'utilisation de l'EDTA est de plus en plus décriée, notamment à cause de l'écotoxicité de la forme complexée de cet agent.

De surcroît, le nombre d'antioxydants disponibles en cosmétique se réduit progressivement puisque plusieurs antioxydants traditionnellement utilisés en cosmétique sont soupçonnés d'être nocifs pour l'homme ou l'environnement. C'est le cas notamment de l'hydroxyanisole butylé (BHA) et de l'hydroxytoluène butylé (BHT), qui s'avèrent être des antioxydants synthétiques largement employés en cosmétique mais soupçonnés d'être écotoxiques, allergisants et d'être des perturbateurs endocriniens ainsi que potentiellement cancérigènes.

A la connaissance du Demandeur, le métabisulfite de sodium peut être utilisé pour la stabilisation des polyphénols et gallates.

A titre d'exemple, WO 2008/052674 décrit une méthode de stabilisation et de réduction de la décoloration du polyphénol via l'utilisation d'un sel de métabisulfite, de préférence le métabisulfite de sodium, notamment pour la prévention et le traitement des pathologies dentaires.

DE 10 2009 048974 décrit des compositions cosmétiques contenant des composés actifs peu solubles dans huile, en l'espèce, des polyphénols, et un agent antioxydant utilisé pour stabiliser la composition tel que le métabisulfite de sodium.

Toutefois, cette utilisation n'est pas toujours suffisante pour conserver la stabilité des actifs contenus dans les produits cosmétiques, afin qu'ils restent inaltérés tout au long de leur temps d'utilisation.

En parallèle, d'autres agents sont mis en oeuvre pour tenter de stabiliser des compositions.

Par exemple, EP 1 166 780 décrit des préparations cosmétiques ou dermatologiques ayant un pH inférieur 5 et contenant des acides sulfiniques, dont l'hypotaurine, pour capter les radicaux libres.

US 2011/151077 décrit l'utilisation d'agents antioxydants, dont l'hypotaurine, dans le but de diminuer ou d'éviter le brunissement des aliments.

FR 3 007 652 décrit une composition cosmétique comprenant du pentaerythritol tetrakis en tant qu'antioxydant et pour application topique sur les matières kératiniques.

Il subsiste donc un besoin évident d'identifier des solutions alternatives, écocompatibles et non toxiques pour l'homme, permettant de combattre efficacement la dégradation des ingrédients actifs instables utilisés en cosmétique, en particulier les composés phénoliques, de préférence les polyphénols et les gallates, avantageusement le gallate de propyle.

Ainsi, le problème que se propose de résoudre l'invention est de fournir une composition simple, non toxique pour l'homme et l'environnement, apte à stabiliser au moins un actif dans une composition cosmétique, pour application topique. De manière générale, la présente invention vise une composition cosmétique stabilisante qui ne présente pas les inconvénients mentionnés précédemment.

## DESCRIPTION DE L'INVENTION

De manière inattendue, le Demandeur a constaté que la combinaison de métabisulfite de sodium et d'hypotaurine présentait une activité stabilisante améliorée vis-à-vis des actifs instables contenus dans les compositions cosmétiques.

Plus particulièrement, le Demandeur a mis au point une composition permettant d'améliorer la stabilité d'au moins un actif dans une composition cosmétique, en combinant au moins du métabisulfite de sodium et de l'hypotaurine.

Au sens de la présente invention, on désigne par « actif instable », toute molécule pourvue d'une activité dermocosmétique et qui soit susceptible de se dégrader au cours du temps de stockage et/ou en réponse à une exposition aux rayons ultraviolets (UV), à la température (supérieure ou inférieure à la température ambiante, environ 25°C), ou à d'autres facteurs (présence d'ions métalliques, pH, réaction avec d'autres constituants des produits etc).

Au sens de l'invention, on désigne par « stabilité des actifs cosmétiques », la stabilité physicochimique des molécules actives, c'est-à-dire, la mesure dans laquelle lesdites molécules sont protégées de l'oxydation, la dimérisation, la polymérisation, l'agrégation, la carbonylation, la glycation ou toute autre forme de modification de la structure de la molécule. Cette stabilité de l'actif cosmétique est différente de la stabilité de la forme galénique du produit, qui est en général indépendante des actifs.

En pratique, l'instabilité de l'actif peut être déterminée par mesure de l'oxydation en formulation.

En pratique, la stabilité d'un actif peut être mesurée à l'aide d'un colorimètre, par exemple le colorimètre (CM-700d/600d Konica Minolta). Cet appareil permet de mesurer et de comparer les couleurs de deux ou plus échantillons/formulations de façon quantitative. Plus particulièrement, il est apte à caractériser le jaunissement ou le brunissement des compositions cosmétiques.

Selon l'invention, la mesure du paramètre de colorimétrie s'effectue dans un milieu test correspondant avantageusement à une base de préférence neutre. Cette base correspond à des assemblages simples de matières premières qui apparaissent blanc à l'oeil nu et qui ne changent pas de couleur en réponse aux stress (UV, lumière, température etc)

En détail, le colorimètre est accompagné d'un logiciel permettant de restituer les variations de la couleur selon la convention CIE L*a*b*, adoptée par la Commission Internationale de l'Eclairage (CIE) depuis 1976. Selon cette convention, les unités de mesure L*a*b* sont des coordonnées colorimétriques et définissent un espace de couleur pour la caractérisation des surfaces où:

- L* représente la clarté, allant du noir au blanc ;
- a* représente l'axe rouge et l'axe vert en passant par le gris ;
- b* représente l'axe jaune et l'axe bleu en passant par le gris.

Enfin, on peut interpréter la valeur ΔE*ab comme la mesure de différence entre la couleur de deux formulations conformément à l'équation suivante :

$$\Delta E * ab = \sqrt{(L *_2 - L *_1)^2 + (a *_2 - a *_1)^2 + (b *_2 - b *_1)^2}$$

Où L*1, a*1, b*1 correspondent aux coordonnées colorimétriques de la première formulation à comparer à L*2, a*2, b*2 correspondant aux coordonnées colorimétriques de la seconde formulation.

Une valeur de ΔE*ab inférieure à 1 signifie que la couleur de la formulation se rapproche de celle de la formule témoin. Une valeur théorique de ΔE*ab égale à 0 indique que les couleurs des deux formulations sont parfaitement identiques. Les actifs stables au sens de l'invention, avantageusement dans la base neutre dans laquelle ils sont incorporés, sont ceux dont la valeur ΔE*ab est inférieure ou égale à 1. Enfin, une valeur de ΔE*ab supérieure à 1 indique que la formulation a subi une variation de couleur significative. Les actifs instables au sens de l'invention, avantageusement dans la base neutre dans laquelle ils sont incorporés, sont ceux dont la valeur ΔE*ab est supérieure à 1.

Selon un premier aspect, la présente invention concerne une composition cosmétique pour application topique comprenant les composés suivants:

- hypotaurine (acide 2-aminoéthanesulfinique) représentant entre 0,001% et 1% en poids total de la composition ;
- métabisulfite de sodium, représentant entre 0,001% et 1% en poids total de la composition ; et
- au moins un actif instable choisi dans le groupe comprenant les polyphénols et les gallates.

L'acide 2-aminoéthanesulfinique, plus communément appelé hypotaurine, est un acide sulfinique intermédiaire de la biosynthèse de la taurine. L'hypotaurine et la taurine agissent chez l'homme comme des agonistes partiels endogènes qui se lient sur le site de fixation de la glycine, notamment des récepteurs à canal chlore tel que les récepteurs GABAergiques. Il s'agit donc d'une molécule endogène parfaitement sûre pour son utilisation cosmétique chez l'homme.

En pratique, dans la composition cosmétique selon l'invention, on peut utiliser une forme pure ou hautement purifiée d'hypotaurine. De préférence, l'hypotaurine est obtenue par synthèse chimique. A titre d'exemple, on peut citer la matière première cosmétique hypotaurine correspondant à la dénomination INCI aminoethanesulfinic acid et commercialisée par l'entreprise DKSH.

En pratique, l'hypotaurine représente entre 0,001% et 1% en poids total de la composition, de préférence entre 0,01% et 0,1%.

Le métabisulfite de sodium, connu sous la dénomination E223, est utilisé comme agent de conservation et antioxydant, notamment dans le domaine de l'alimentation. Il s'agit d'une molécule sûre pour son utilisation cosmétique chez l'homme. En effet, le métabisulfite de sodium n'a pas d'effets secondaires identifiés. En cas de pénétration dans le corps humain, il est oxydé par le foie en sulfate, inoffensif, et excrété dans l'urine.

En pratique, dans la composition selon l'invention, on peut utiliser une forme pure ou hautement purifiée de métabisulfite de sodium, de préférence d'origine chimique. A titre d'exemple, on peut citer la matière première cosmétique métabisulfite de sodium correspondant à la dénomination INCI sodium métabisulfite et commercialisée par l'entreprise COOPER INDUSTRIES.

En pratique, le métabisulfite de sodium représente entre 0,001% et 1% en poids total de la composition, de préférence entre 0,01% et 0,5%, encore plus avantageusement entre 0,1% et 0,2%.

Dans un mode de réalisation particulier selon l'invention, la composition comprend en outre du pentaerythritol tetrakis (3,5-di-tert-butyl-4-hydroxyhydrocinnamate). Le Demandeur a constaté que de manière inattendue, cette molécule agit avec le métabisulfite de sodium et l'acide 2-aminoéthanesulfmique pour améliorer ou rétablir la stabilité des actifs instables au cours du temps, de préférence estomper ou abolir les modifications de couleur de la composition cosmétique contenant lesdits actifs instables, notamment les polyphénols ou les gallates.

Le pentaerythritol tetrakis (3,5-di-tert-butyl-4-hydroxyhydrocinnamate) est notamment connu et utilisé pour ses propriétés antioxydantes, en particulier dans les huiles pour prévenir la détérioration des caractéristiques organoleptiques.

Il s'agit d'une matière première considérée sûre pour son utilisation cosmétique chez l'homme. A titre d'exemple, la matière première commercialisée par la société BASF sous la dénomination Tinogard TT et correspondant à la désignation INCI pentaerythrityl tetra-di-t-butyl-hydroxyhydrocinnamate peut être utilisée dans la composition selon l'invention.

Selon l'invention, le pentaerythritol tetrakis représente avantageusement entre 0,001% et 1% en poids total de la composition, de préférence entre 0,01% et 0,1%.

Ainsi, et selon un mode de réalisation avantageux, la présente invention concerne une composition cosmétique pour application topique comprenant les composés suivants:

- hypotaurine (acide 2-aminoéthanesulfinique), représentant entre 0,001% et 1% en poids total de la composition ;
- métabisulfite de sodium, représentant entre 0,001% et 1% en poids total de la composition ;
- pentaerythritol tetrakis (3,5-di-tert-butyl-4-hydroxyhydrocinnamate) ; et
- au moins un actif instable choisi dans le groupe comprenant les polyphénols et les gallates.

En pratique, selon la présente invention, une composition cosmétique stable pour application topique comprend en outre, au moins un actif instable.

Dans un mode de réalisation particulier, l'actif est une molécule pure, hautement purifiée ou bien, contenue dans un extrait végétal.

Selon un mode de réalisation particulier, l'actif instable est un composé phénolique choisi dans le groupe comprenant les polyphénols et les gallates.

La composition cosmétique selon l'invention comprend de préférence un actif instable tel que décrit précédemment, représentant avantageusement entre 0,0001% et 20% en poids total de la composition, de préférence entre 0,0001% et 10%, plus avantageusement entre 0,01% et 1%.

Dans un mode de réalisation particulier, l'actif est un polyphénol.

Les polyphénols sont des molécules caractérisées par la présence de plusieurs groupements phénoliques associés en structure plus ou moins complexe généralement de haut poids moléculaire. Ce sont des composés généralement hydrosolubles, de poids moléculaire compris entre 50 et 3000 daltons. La famille des polyphénols est communément subdivisée en sous-familles qui sont les tannins, les lignines, les flavonoïdes et les anthocyanes et qui dérivent tous de l'assemblement de simples unités phénoliques.

Dans la composition selon l'invention, les polyphénols représentent avantageusement entre 0,001% et 10% en poids total de la composition, de préférence entre 0,005% et 5%.

Dans un mode de réalisation particulier, l' actif est un flavonoïde. Les flavonoïdes constituent différentes classes chimiques, parmi lesquelles on peut citer les flavanones, les flavones, les flavonols, les dihydroflavonols, les catéchines, les leucoanthocyanidines, les chalcones, les aurones et les dihydrochalcones. Ces flavonoïdes sont connus et notamment décrits dans l'ouvrage « the flavonoids » (Harborne J.B., Mabry T.J., Helga Mabry, 1975, pages 1 à 45). Dans un mode de réalisation particulier, le flavonoïde est choisi dans le groupe comprenant les flavanones, les flavones, les flavonols, les dihydroflavonols, les catéchines et les leucoanthocyanidines.

Les tanins constituent une classe de polyphénols complexes construits par des unités d'acide gallique (gallotanins), ou encore par des unités de flavones (oligomères ou polymères de flavanols, plus communément appelés tanins condensés), ou de phloroglucinol (phlorotanins). Typiquement, les polyphénols nécessitent au moins douze groupes hydroxyle et au moins cinq groupes phényle pour être considérés comme des tanins.

Dans un mode de réalisation particulier, l'actif est un tanin.

Selon l'invention, le polyphénol est un flavonoïde, de préférence ledit flavonoïde est choisi dans le groupe comprenant la catéchine, l'épicatéchine, la naringénine, la rutine, la troxérutine, la dihydromyricetine, la nicotiflorine, l'épigallocatéchine, le gallate de l'épigallocatéchine, la quercetine et la génistéine.

Dans un autre mode de réalisation particulier selon l'invention, l'actif est un gallate. Les gallates sont les dérivés monophénoliques de l'acide gallique, ou acide 3,4,5-trihydroxybenzoïque. Les gallates possèdent des propriétés intéressantes d'un point de vue dermocosmétique. A titre d'exemple, le document FR 2 857 266 met en évidence l'activité sébofluidifiant de plusieurs gallates, qui les rendent particulièrement efficaces dans le traitement des comédons et des peaux grasses.

Cependant, les gallates sont susceptibles de s'oxyder dans les compositions cosmétiques, surtout dans des conditions de pH supérieur à 5,5 ou inférieur à 4, et/ou en présence d'ions métalliques. L'oxydation des gallates cause le jaunissement ou brunissement de formule, ce qui empêche leur utilisation dans les formules non teintées, du moins à des doses actives.

Selon l'invention, l'association d'hypotaurine (acide 2-aminoéthanesulfinique) et métabisulfite de sodium, chacun représentant entre 0,001% et 1% en poids total de la composition, avantageusement supplémentée avec du pentaerythritol tetrakis (3,5-di-tert-butyl-4-hydroxyhydrocinnamate), stabilise efficacement et durablement un actif dit instable. La stabilisation de concentrations significatives de l'actif permet d'obtenir et de proposer à la vente des produits contenant des doses plus importantes d'actifs, et donc des produits plus efficaces.

Selon l'invention, le gallate est choisi dans le groupe comprenant le gallate de propyle, le gallate d'octyle, le gallate de dodécyle, de préférence le gallate de propyle.

Selon un mode de réalisation particulier de l'invention, les gallates représentent avantageusement entre 0,0001% et 20% en poids total de la composition, de préférence entre 0,0001% et 10%, encore plus avantageusement entre 0,01% et 1%.

A titre d'exemple, les matières premières cosmétiques OriStar PG (INCI propyl gallate), OriStar DG (INCI dodecyl gallate), commercialisées par ORIENT STARS LLC et Octyl gallate, commercialisée par LG HOUSEHOLD & HEALTH CARE LTD. peuvent être utilisées comme sources de gallates.

Un autre aspect de la présente invention concerne l'utilisation de l'association d'hypotaurine (acide 2-aminoéthanesulfinique), de métabisulfite de sodium, éventuellement supplémentée avec du pentaerythritol tetrakis (3,5-di-tert-butyl-4-hydroxyhydrocinnamate) pour réduire ou abolir la dégradation d'au moins un actif instable contenu dans une composition cosmétique, en d'autres termes, améliorer la stabilité d'au moins un actif, ledit actif instable est choisi dans le groupe comprenant les polyphénols ou les gallates, de préférence l'actif instable selon l'invention est le gallate de propyle.

Dans un mode de réalisation préféré, la composition selon l'invention est formulée avec une eau de formulation correspondant à une solution aqueuse saline et minéralisée répondant à au moins deux paramètres bioélectriques ou bioélectriques, dont l'un est le potentiel redox, et dont l'autre est choisi entre le pH et la résistivité. Cette eau est en équilibre électronique avec l'eau cellulaire et donc particulièrement adaptée à l'application topique.

Ainsi, préférentiellement, au moins deux paramètres bioélectriques de l'eau de formulation de la composition selon l'invention, dont l'un est le potentiel redox, et dont l'autre est choisi entre le pH et la résistivité, sont réglés par la composition en sels minéraux qu'elle contient, pour avoir une valeur:

- comprise entre 5 et 8, en ce qui concerne le pH ;
- comprise entre 10 et 29, en ce qui concerne le potentiel redox ;
- et une résistivité substantiellement comprise entre 8000 ohms.cm et 80 ohms.cm.

Dans un mode de réalisation préféré, l'eau de formulation de la composition selon l'invention possède une conductivité substantiellement comprise entre 125 et 12500 μS/cm.

Avantageusement, la pression osmotique de l'eau de formulation selon l'invention est réglée par sa composition minérale, pour avoir une valeur comprise entre 70 et 1500 mOsmole, de préférence entre 200 et 400 mOsmole, par exemple comprise entre 280 et 320 mOsmole.

A titre d'exemple, ce sont les trois paramètres bioélectriques de l'eau de formulation définie précédemment, qui sont réglés par la composition minérale, pour avoir les valeurs définies ci-dessus, respectivement pour le pH, le potentiel redox et la résistivité. L'eau de formulation peut donc comprendre par exemple des adjuvants ou tampons permettant de régler ou ajuster son pH.

Dans un mode de réalisation particulier, la composition selon l'invention comprend en outre un système bioactif associant, d'une part une forme stable en solution aqueuse d'ATP (adénosine-triphosphate) avec éventuellement un précurseur d'ATP, par exemple Gp4G (diguanosine tétraphosphate) ou Ap4A (diadénosine tétraphosphate), et d'autre part au moins un peptide biomimétique comprenant au plus six acides aminés, mimant un polypeptide cutané ou une protéine cutanée, ou une biomolécule agoniste ou antagoniste audit peptide ou à ladite protéine. Conformément aux divulgations du document EP 1 581 177, l'association de ces principes actifs permet d'augmenter l'activité métabolique des cellules de la peau tout en obtenant un effet dermocosmétique ou thérapeutique grâce à l'utilisation des peptides biomimétiques. Ces peptides biomimétiques peuvent être sélectionnés afin d'obtenir l'effet souhaité, par exemple, un effet d'inhibition des irritations d'origine neurogène, une activité dépigmentante, un effet inhibant toute intolérance ou sensibilisation etc.

En pratique, dans le système bioactif selon l'invention, l'ATP et éventuellement le précurseur d'ATP représentent au plus 10% en poids total de la composition, de préférence entre 0,001 % et 5% et le peptide biomimétique entre 0,001% à 1% en poids total de la composition.

La composition cosmétique selon l'invention peut également comprendre les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants autres que ceux de l'invention, les parfums, les charges, les pigments, les filtres UV, les absorbeurs d'odeur, les colorants, les hydratants, des vitamines, des acides gras essentiels, des polymères liposolubles notamment hydrocarbonés, les opacifiants, les stabilisants, les séquestrants, les conditionneurs, les agents propulseurs, les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), colorants, les agents nacrants, les filtres solaires et notamment les filtres sulfoniques, les parfums, les conservateurs, les agents antimicrobiens, les électrolytes, les agents stabilisants tels que l'acide érythorbique.

La composition peut se présenter sous forme de lotion aqueuse ou hydroalcoolique, monophasique ou polyphasique, de gel monophasique ou polyphasique, d'émulsion huile dans eau (H/E), eau dans huile (E/H), eau dans silicone (E/S), silicone dans eau (S/E), ou encore sous forme d'émulsion triple, de dispersion vésiculaire, de mousse, de pulvérisation (spray), de crème, de brume, de solution ou de lait.

Plus précisément, parmi les corps gras, on désigne une huile ou une cire ou leur mélange, des acides gras, des alcools gras, des esters d'acides gras tels que les triglycérides d'acide gras en $C_6$ à $C_{18}$, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone, les isoparaffines et les huiles fluorées ou perfluorées.

Parmi les cires, on peut citer les cires animales, végétales, minérales ou de synthèse, et notamment les cires d'abeilles, de Candelilla, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en $C_1$ à $C_6$ comme l'éthanol, l'isopropanol, l'éthylèneglycol, le diéthylènegycol, le propylèneglycol ou le glycérol.

Les agents épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, la gomme de xanthane, les scléroglucanes, les acides polyacryliques réticulés.

Le pH de la composition selon l'invention est généralement compris entre 3 et 9, de préférence entre 3 et 6.

Bien entendu, l'homme du métier veillera à choisir ce, ou ces éventuels adjuvants ou excipients complémentaires, et/ou leur quantité, de manière telle que les propriétés avantageuses de la composition selon l'invention ne soient pas, ou substantiellement pas, altérées par l'adjonction envisagée.

La manière dont l'invention peut être réalisée et les avantages qui en découlent, ressortiront mieux des exemples de réalisation qui suivent, donnés à titre indicatif et non limitatif.

**EXEMPLES DE REALISATION**

Les pourcentages correspondent au poids du produit indiqué par rapport au poids total de la composition.

**Exemple I - Crème visage - émulsion E/S** (hors invention)

| Ingrédient (INCI) | % |
|---|---|
| Aqua/water/eau | Qsp 100 |
| |Methyl méthacrylate crosspolymer | 7,750000 |
| Dipropylene glycol | 5,000000 |
| Cyclopentasiloxane | 4,720000 |
| Cyclohexasiloxane | 3,800000 |
| Dimethicone | 2,070000 |
| Glycerin | 2,000000 |
| Butylene glycol | 1,527000 |
| *Fomes officinalis* (mushroom) extract | 1,440000 |
| Tocopheryl acetate | 1,000000 |
| Sodium polyacrylate | 0,780000 |
| Pentylene glycol | 0,500360 |
| Silica | 0,500000 |
| Lauryl PEG/ppg-18/18 methicone | 0,450000 |
| 1,2-hexanediol | 0,250000 |
| C30-45 alkyl cetearvl dimethicone crosspolymer | 0,250000 |
| Caprylyl glycol | 0,250000 |
| Creatine | 0,200000 |
| Sodium citrate | 0,200000 |
| *Candida bombicolalglucose*/*methyl* rapeseedate ferment | 0,180000 |
| Propylene glycol | 0,142860 |
| Pentaerythrityl tetra-di-t-butyl hydroxyhydrocinnamate | 0,100000 |
| Trideceth-6 | 0,090000 |
| Aminoethanesulfinic acid | 0,050000 |
| Isostearyl alcohol | 0,050000 |
| Peg/ppg-18/18 dimethicone | 0,030000 |
| Phenoxyethanol | 0,024096 |
| Fragrance (parfum) | 0,020000 |
| Peg-40 hydrogenated castor oil | 0,009000 |
| *Ginkgo biloba* leaf extract | 0,008000 |
| Carnosine | 0,006406 |
| Propyl gallate | 0,005000 |
| Disodium adenosine triphosphate | 0,003203 |

(suite)

| Ingrédient (INCI) | % |
|---|---|
| Potassium sorbate | 0,002400 |
| Laminaria digitata extract | 0,001730 |
| Superoxide dismutase | 0,000066 |
| Tromethamine | 0,000066 |

**Exemple II -Crème visage - émulsion H/E**

| Ingrédient | % |
|---|---|
| Aqua/water/eau | Qsp 100 |
| Cyclopentasiloxane | 6,150000 |
| Methyl méthacrylate crosspolymer | 5,000000 |
| Dipropylene glycol | 4,000000 |
| Isononyl isononanoate | 4,000000 |
| Dimethicone | 3,000000 |
| Zinc gluconate | 3,000000 |
| Cyclohexasiloxane | 2,850000 |
| Arachidyl alcohol | 1,650000 |
| Butylene glycol | 1,530000 |
| Cetyl alcohol | 1,500000 |
| Glyceryl stéarate | 1,000000 |
| PEG-100 stéarate | 1,000000 |
| Tocopheryl acetate | 1,000000 |
| Behenyl alcohol | 0,900000 |
| Sodium acrylates copolymer | 0,798000 |
| Hydrogenated polyisobutene | 0,600000 |
| Pentylene glycol | 0,500360 |
| Arachidyl glucoside | 0,450000 |
| 1,2-hexanediol | 0,250000 |
| ICaprylyl glycol | 0,250000 |
| Creatine | 0,200000 |
| Helianthus annuus (sunflower) seed oil | 0,200000 |
| Phospholipids | 0,200000 |
| Polyglyceryl-10 stéarate | 0,200000 |
| Salicylic acid | 0,200000 |
| Candida bombicolalglucose/methyl rapeseedate ferment | 0,180000 |
| Fragrance (parfum) | 0,150000 |
| Sodium metabisulfite | 0,150000 |

(suite)

| Ingrédient | % |
|---|---|
| Propylene glycol | 0,134870 |
| Sodium hydroxide | 0,080000 |
| Pentaerythrityl tetra-di-t-butyl hydroxyhydrocinnamate | 0,050000 |
| Pyridoxine HCl | 0,050000 |
| Dodecyl gallate | 0,030000 |
| Aminoethanesulfinic acid | 0,025000 |
| Phenoxyethanol | 0,024096 |
| *Fomes officinalis* (mushroom) extract | 0,024000 |
| PEG-40 hydrogenated castor oil | 0,009000 |
| *Ginkgo biloba* leaf extract | 0,008000 |
| Carnosine | 0,005565 |
| Algae extract | 0,002783 |
| Disodium adenosine triphosphate | 0,002783 |
| Potassium sorbate | 0,002400 |
| Tocopherol | 0,002000 |
| Superoxide dismutase | 0,000066 |
| Tromethamine | 0,000066 |

**Exemple III- Stabilisation de l'actif gallate de propyle par l'association d'antioxydants selon l'invention**

La base la plus neutre possible, c'est-à-dire blanche, et la plus simple en terme de composition, a été choisie pour les tests. Il s'agit ici, d'une base de test sous forme d'une émulsion gel-crème. Au préalable, des gammes de concentration ont été effectuées pour mettre en évidence le jaunissement/brunissement du gallate de propyle dans cette base.

La dose de 0,1% en poids total de la composition de gallate de propyle a été retenue pour les essais comparatifs.

Les formules suivantes ont été préparées :

| Ingrédient (INCI) | Stockage à 4°C | Formules soumises à un cycle de SUNTEST | | | | | |
|---|---|---|---|---|---|---|---|
| | T (%) | EC1 (%) | EC2 (%) | EC3 (%) | EC4 (%) | EC5 (%) | EC6 (%) |
| Aqua/water/eau | 87,925 | 87,775 | 87,90 | 87,875 | 87,75 | 87,725 | 87,70 |
| Caprylic/capric triglycéride | 5,00 | 5,00 | 5,00 | 5,00 | 5,00 | 5,00 | 5,00 |
| Butylene glycol | 3,00 | 3,00 | 3,00 | 3,00 | 3,00 | 3,00 | 3,00 |
| Hydroxyethyl acrylate/sodium acryloyldimethyl taurate copolymer | 1,85 | 1,85 | 1,85 | 1,85 | 1,85 | 1,85 | 1,85 |
| Squalane | 1,275 | 1,275 | 1,275 | 1,275 | 1,275 | 1,275 | 1,275 |
| Phenoxyethanol | 0,5 | 0,5 | 3,5 | 0,5 | 0,5 | 0,5 | 0,50 |
| Polysorbate 60 | 0,275 | 0,275 | 0,275 | 0,275 | 0,275 | 0,275 | 0,275 |
| Sodium metabisulfite | - | 0,15 | - | - | 0,15 | 0,15 | 0,15 |

(suite)

| Ingrédient (INCI) | Stockage à 4°C | Formules soumises à un cycle de SUNTEST | | | | | |
|---|---|---|---|---|---|---|---|
| | T (%) | EC1 (%) | EC2 (%) | EC3 (%) | EC4 (%) | EC5 (%) | EC6 (%) |
| Propyl gallate | 0,1 | 0,1 | 0,1 | 0,1 | 0,1 | 0,1 | 0,10 |
| Sorbitan isostearate | 0,075 | 0,075 | 0,075 | 0,075 | 0,075 | 0,075 | 0,075 |
| Pentaerythrityl tetra-di-t-butyl hydroxyhydrocinnamate | - | - | - | 0,05 | - | 0,05 | 0,05 |
| Aminoethanesulfinic acid | - | - | 0,025 | - | 0,025 | - | 0,025 |

La formule témoin « T » ne contient aucun des antioxydants selon l'invention. Le couleur de formule à l'origine est blanc-ivoire. Lorsque cette formule est conservée à 4°C, aucune modification de couleur n'est détectée à l'oeil nu.

Cette formule stockée à 4°C a été choisie comme témoin de référence par la suite.

Après exposition à différents stress, soit à la lumière en utilisant un appareillage « Suntest », soit après incubation en étuve à 80°C pendant 1 semaine, la composition présente un brunissement inacceptable.

Les antioxydants selon l'invention pris individuellement et leurs associations ont été mis en oeuvre dans les essais comparatifs « EC1-EC6 ».

Afin de quantifier précisément l'ampleur de jaunissement/brunissement dans la formule témoin ainsi que l'effet des associations d'antioxydants selon l'invention, des mesures colorimétriques ont été effectuées.

Le colorimètre (CM-700d/600d Konica Minolta) permet de mesurer et de comparer les couleurs des formules de façon quantitative. L'appareillage est composé d'une source de lumière d'intensité variable, d'un dispositif optique pour la focalisation et l'orientation de la lumière, d'un dispositif permettant la séparation et l'isolement des différentes radiations extérieures, et d'un dispositif de mesure de l'énergie lumineuse à l'entrée et à la sortie de la cuve.

La détection de la couleur des échantillons est basée sur l'étalon colorimétrique D65 correspondant à une lumière naturelle en plein jour en zone tempérée. Il s'agit d'un blanc froid qui correspond à une température de couleur proximale de 6500K.

Le colorimètre est accompagné du logiciel «SpectraMagicNX» permettant de restituer les variations de la couleur de l'échantillon selon la convention CIE L*a*b*, adoptée par la Commission internationale de l'éclairage (CIE) depuis 1976. Selon cette convention, les unités de mesure L*a*b* définissent un espace de couleur pour la caractérisation des surfaces :

- L* représente la clarté, du noir au blanc.
- a* représente l'axe rouge et l'axe vert en passant par le gris.
- b* représente l'axe jaune et l'axe bleu en passant par le gris.
- Enfin, on peut interpréter la valeur ΔE*ab comme la mesure de différence entre la couleur de deux échantillons, conformément à l'équation suivante :

$$\Delta E*ab = \sqrt{(L*_2 - L*_1)^2 + (a*_2 - a*_1)^2 + (b*_2 - b*_1)^2}$$

Où L*1, a*1, b*1 correspondent aux coordonnées colorimétriques CIE LAB de la première formule, qui sont comparées à L*2, a*2, b*2, correspondant aux coordonnées colorimétriques de la seconde formule.

Une valeur de ΔE*ab inférieure à 1 signifie que la couleur de la formule testée se rapproche de celle de la formule témoin. Une valeur théorique de ΔE*ab égale à 0 signifie que la couleur des deux échantillons est parfaitement identique. Enfin, une valeur de ΔE*ab supérieure à 1 signifie que la formule a subi une variation de couleur très significative.

Les compositions T et EC1 à EC6 subissent un cycle de Suntest, (appareillage : Suntest XLS+ filtre FG (Filter Glass) - ATLAS MATERIAL TESTING SOLUTIONS ; Protocole : 24H à 45-50°C à une puissance de 730W/m²), et leurs coordonnées colorimétriques sont comparées à celles de la composition de référence T avant aaplication d'un cycle de Suntest.

| Fle | Suntest | ΔE*ab(D65) | L*(D65) | Axe a* | Axe b* |
|---|---|---|---|---|---|
| T | Non | --- | --- | --- | --- |

(suite)

| T | Oui | 11,26 | Plus foncé | Plus vert | Plus jaune |
|---|-----|-------|------------|-----------|------------|
| EC1 | Oui | 0,52 | plus foncé | moins vert | moins jaune |
| EC2 | Oui | 9,82 | plus foncé | plus vert | plus jaune |
| EC3 | Oui | 6,68 | plus foncé | plus vert | plus jaune |
| EC4 | Oui | 0,38 | plus foncé | moins vert | moins jaune |
| EC5 | Oui | 1,02 | plus foncé | moins vert | moins jaune |
| EC6 | Oui | 0,29 | plus clair | moins vert | moins jaune |

Les résultats montrent qu'en absence d'antioxydants, la composition T subit un changement de couleur inacceptable ($\Delta E^*ab$ = 11,26).

Parmi les antioxydants seuls, seulement le métabisulfite de sodium permet d'obtenir une valeur de $\Delta E^*ab$ inférieure à 1 (EC1).

De manière inattendue, l'association du métabisulfite de sodium avec l'hypotaurine améliore la stabilité du gallate de propyle (EC4). En effet, la valeur de $\Delta E^*ab$ est de 0,38, soit inférieure à celle du métabisulfite seul (EC1 ; $\Delta E^*ab$= 0,52) et largement inférieure à celle de l'hypotaurine seul (EC2 ; $\Delta E^*ab$= 9,82).

De façon toute aussi inattendue, l'association d'hypotaurine, de métabisulfite de sodium et de pentaerythrityl tetra-di-t-butyl hydroxyhydrocinnamate (EC6) permet d'obtenir la meilleure réduction de la coloration sur les trois axes (L*, a*, b*) ainsi que la valeur de $\Delta E^*ab$ la plus faible ($\Delta E^*ab$= 0,29).

Ces données indiquent que la composition a subi la variation de couleur la plus faible par rapport au témoin T, après une exposition au rayons UV (Suntest).

En conclusion, la composition selon l'invention comprenant au moins un actif instable et une association d'au moins deux antioxydants, c'est-à-dire l'hypotaurine et le métabisulfite de sodium, éventuellement supplémentée par le pentaerythritol tetrakis (3,5-di-tert-butyl-4-hydroxyhydrocinnamate), permet de stabiliser l'actif. Cette stabilisation a pour conséquence de ne pas modifier la couleur de la composition (jaunissement ou brunissement) suite à une exposition aux rayons UV.

**Revendications**

1. Composition cosmétique pour application topique comprenant les composés suivants :

   - hypotaurine (acide 2-aminoéthanesulfinique), représentant entre 0,001% et 1% en poids total de la composition ; et
   - métabisulfite de sodium, représentant entre 0,001% et 1% en poids total de la composition ; et
   - au moins un actif instable choisi dans le groupe comprenant les polyphénols et les gallates.

2. Composition cosmétique selon la revendication 1, **caractérisée en ce qu'**elle comprend en outre le pentaerythritol tetrakis (3,5-di-tert-butyl-4-hydroxyhydrocinnamate).

3. Composition cosmétique selon la revendication 1 ou 2, **caractérisée en ce que** le polyphénol est un flavonoïde, de préférence choisi dans le groupe comprenant la catéchine, l'épicatéchine, la naringénine, la rutine, la troxérutine, la dihydromyricetine, la nicotiflorine, l'épigallocatéchine, le gallate de l'épigallocatéchine, la quercetine et la génistéine.

4. Composition cosmétique selon l'une quelconque des revendications précédentes, **caractérisée en ce que** le gallate est choisi dans le groupe comprenant le gallate de propyle, le gallate d'octyle, le gallate de dodécyle, de préférence le gallate de propyle.

5. Composition selon l'une quelconque des revendications précédentes, **caractérisée en ce que** l'hypotaurine représente entre 0,01% et 0,1% en poids total de la composition.

6. Composition cosmétique selon l'une quelconque des revendications précédentes, **caractérisée en ce que** le métabisulfite de sodium représente entre 0,01% et 0,5% en poids total de la composition, avantageusement entre 0,1% et 0,2%.

7. Composition cosmétique selon l'une quelconque des revendications précédentes, **caractérisée en ce que** le pentaerythritol tetrakis (3,5-di-tert-butyl-4-hydroxyhydrocinnamate) représente entre 0,001% et 1% en poids total de la composition, de préférence entre 0,01% et 0,1%.

8. Composition cosmétique selon l'une des revendications 3 à 7, **caractérisée en ce que** le au moins un actif instable, représente entre 0,0001% et 20% en poids total de la composition, de préférence entre 0,0001% et 10%, avantageusement entre 0,01% et 1%.

9. Utilisation d'hypotaurine et de métabisulfite de sodium, éventuellement supplémentés avec du pentaerythritol tetrakis (3,5-di-tert-butyl-4-hydroxyhydrocinnamate), pour stabiliser au moins un actif instable contenu dans une composition cosmétique, ledit actif instable est choisi dans le groupe comprenant les polyphénols et les gallates.

## Patentansprüche

1. Kosmetische Zusammensetzung zur äußerlichen Anwendung, die die folgenden Verbindungen umfasst:

   - Hypotaurin (2-Aminoethansulfinsäure), das zwischen 0,001 % und 1 % des Gesamtgewichts der Zusammensetzung darstellt; und
   - Natriummetabisulfit, das zwischen 0,001 % und 1 % des Gesamtgewichts der Zusammensetzung darstellt; und
   - zumindest einen instabilen Wirkstoff, gewählt in der Gruppe, die Polyphenole und Gallate umfasst.

2. Kosmetische Zusammensetzung nach Anspruch 1, **dadurch gekennzeichnet, dass** sie weiterhin Pentaerythritol-tetrakis (3,5-di-tert-butyl-4-hydroxyhydrocinnamat) umfasst.

3. Kosmetische Zusammensetzung nach Anspruch 1 oder 2, **dadurch gekennzeichnet, dass** das Polyphenol ein Flavonoid ist, das bevorzugt gewählt ist in der Gruppe, die Catechin, Epicatechin, Naringenin, Rutin, Troxerutin, Dihydromyricetin, Nicotiflorin, Epigallocatechin, Epigallocatechingallat, Quercetin und Genistein umfasst.

4. Kosmetische Zusammenfassung nach einem der vorstehenden Ansprüche, **dadurch gekennzeichnet, dass** das Gallat gewählt ist in der Gruppe, die Propylgallat, Octylgallat, Dodecylgallat, bevorzugt Propylgallat, umfasst.

5. Zusammensetzung nach einem der vorstehenden Ansprüche, **dadurch gekennzeichnet, dass** Hypotaurin zwischen 0,01 % und 0,1 % des Gesamtgewichts der Zusammensetzung darstellt.

6. Zusammensetzung nach einem der vorstehenden Ansprüche, **dadurch gekennzeichnet, dass** Natriummetabisulfit zwischen 0,01 % und 0,5% des Gesamtgewichts der Zusammensetzung, vorteilhafterweise zwischen 0,1 % und 0,2 %, darstellt.

7. Kosmetische Zusammensetzung nach einem der vorstehenden Ansprüche, **dadurch gekennzeichnet, dass** Pentaerythritol-tetrakis (3,5-di-tert-butyl-4-hydroxyhydrocinnamat) zwischen 0,001 % und 1 % des Gesamtgewichts der Zusammensetzung, bevorzugt zwischen 0,01 % und 0,1 %, darstellt.

8. Kosmetische Zusammensetzung nach einem der Ansprüche 3 bis 7, **dadurch gekennzeichnet, dass** der zumindest eine instabile Wirkstoff zwischen 0,0001 % und 20 % des Gesamtgewichts der Zusammensetzung, bevorzugt zwischen 0,01 % und 1 %, darstellt.

9. Verwendung von Hypotaurin und Natriummetabisulfit, gegebenenfalls ergänzt mit Pentaerythritol-tetrakis (3,5-di-tert-butyl-4-hydroxyhydrocinnamat) zur Stabilisierung zumindest eines in einer kosmetischen Zusammensetzung enthaltenen instabilen Wirkstoffs, wobei besagter instabiler Wirkstoff gewählt ist in der Gruppe, die Polyphenole und Gallate umfasst.

## Claims

1. A cosmetic composition for a topical application, comprising the following compounds:

- hypotaurine (2-aminoethanesulfinic acid), representing between 0.001% and 1% of the total weight of the composition;
- sodium metabisulphite, representing between 0.001% and 1% by total weight of the composition; and
- at least one unstable active agent selected from the group comprising polyphenols and gallates.

2. The cosmetic composition according to claim 1, **characterized in that** it further comprises pentaerythritol tetrakis (3,5-di-tert-butyl-4-hydroxyhydrocinnamate).

3. The cosmetic composition according to claim 1 or 2, **characterized in that** the polyphenol is a flavonoid, preferably chosen from the group comprising catechin, epicatechin, naringenin, rutin, troxerutin, dihydromyricetin, nicotiflorin, epigallocatechin, epigallocatechin gallate, quercetin and genistein.

4. The cosmetic composition according to any one of the preceding claims, **characterized in that** the gallate is selected from the group comprising propyl gallate, octyl gallate, dodecyl gallate, preferably propyl gallate.

5. The cosmetic composition according to any one of the preceding claims, **characterized in that** the hypotaurine represents between 0.01% and 0.1% of the total weight of the composition.

6. The cosmetic composition according to any one of the preceding claims, **characterized in that** the sodium metabisulphite represents between 0.01% and 0.5% of the total weight of the composition.

7. The cosmetic composition according to any one of the preceding claims, **characterized in that** the pentaerythritol tetrakis (3,5-di-tert-butyl-4-hydroxyhydrocinnamate) represents between 0.001% and 1% of the total weight of the composition, preferably between 0.01% and 0.1%.

8. The cosmetic composition according to any one of the preceding claims, **characterized in that** the at least one unstable active agent represents between 0.0001% and 20% of the total weight of the composition, preferably between 0,0001 and 10%, advantageously between 0.01% and 1%.

9. Use of hypotaurin and sodium metabisulfite, optionally supplemented with pentaerythritol tetrakis (3,5-di-tert-butyl-4-hydroxyhydrocinnamate), for stabilizing at least one unstable active agent contained in a cosmetic composition, said unstable active agent being selected from the group comprising polyphenols and gallates.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2778663
- WO 2008052674 A
- DE 102009048974
- EP 1166780 A
- US 2011151077 A
- FR 3007652
- FR 2857266
- EP 1581177 A

**Littérature non-brevet citée dans la description**

- **HARBORNE J.B. ; MABRY T.J. ; HELGA MABRY.** *the flavonoids,* 1975, 1-45